(19) Europäisches Patentamt

European Patent Office

Office européen des brevets

(11) **EP 4 675 238 A1**

(12) **EUROPEAN PATENT APPLICATION**

(43) Date of publication:
07.01.2026 Bulletin 2026/02

(21) Application number: 24185953.7

(22) Date of filing: 02.07.2024

(51) International Patent Classification (IPC):
**G01F 1/66** (2022.01)      **G01F 15/16** (2006.01)
**A61M 16/00** (2006.01)      **A61M 16/08** (2006.01)

(52) Cooperative Patent Classification (CPC):
**A61M 16/00; G01F 1/666; G01F 15/16;**
A61M 16/0875; A61M 2016/003; A61M 2205/3375;
A61M 2205/43

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **OUWELTJES, Okke**
**Eindhoven (NL)**
• **HENDRIKS, Cornelis Petrus**
**Eindhoven (NL)**
• **VAN ZANTEN, Joyce**
**5656AG Eindhoven (NL)**

(74) Representative: **Philips Intellectual Property & Standards**
**High Tech Campus 52**
**5656 AG Eindhoven (NL)**

(54) **RESPIRATORY SUPPORT DEVICE AND METHOD FOR DETECTING AN ACOUSTIC SIGNAL**

(57)     There is provided a respiratory support device for providing pressurized air to a subject. The respiratory support device comprises a conduit, an acoustic generator, and an acoustic sensor. The conduit is for conveying an airflow. The conduit has an opening. The acoustic generator is adapted to generate an acoustic signal in the conduit at an acoustic frequency. The acoustic sensor is arranged outside the conduit. The acoustic sensor is adapted to generate a sensor signal representative of the acoustic signal. The respiratory support device comprises a membrane arranged to cover the opening to separate the acoustic sensor and the airflow from each other. The membrane is adapted to resonate at the acoustic frequency.

FIG. 2

**Description**

FIELD OF THE INVENTION

The invention relates to a respiratory support device for providing pressurized air to a subject. Further, the invention relates to a method for detecting an acoustic signal at an acoustic frequency in a conduit of a respiratory support device providing pressurized air to a subject.

BACKGROUND OF THE INVENTION

Sleep-disordered breathing (SDB) conditions, such as obstructive sleep apnea (OSA) and central sleep apnea (CSA), are becoming increasingly common, and are particularly prevalent in older people, people with a high body mass index, smokers, heavy drinkers and people with conditions such as coronary artery disease, hypertension and diabetes mellitus.

To treat SDB conditions, use is made of respiratory support devices, such as positive airway pressure (PAP) devices. The respiratory support device provides pressurized air to a subject to keep the subject's airways open during sleep. The respiratory support device provides the pressurized air to a subject's nose and/or mouth via a patient interface. Known patient interfaces are a nasal pillow, an oronasal mask, or a full-face mask.

US patent application US 2020/0282161A1 discloses a respiratory therapy system including one or more acoustic generators to produce an inaudible acoustic signal. The acoustic generator, when coupled to a patient interface or an air circuit of a respiratory therapy device, provides inaudible acoustic signals indicative of one or more parameters, such as a flow rate or a pressure of the flow of air, or a type of or useable life of a component (e.g. patient interface). The system has an acoustic sensor that detects one or more acoustic signals from the acoustic generator.

SUMMARY OF THE INVENTION

However, humidified air may flow through the air circuit. The humidified air may damage or contaminate the acoustic sensor. Also, the acoustic sensor may comprise materials that may release particles, such as volatile organic components. Such particles may be harmful to the subject if transferred along the air circuit to the patient interface.

It is an objective of the invention to prevent contamination between the acoustic sensor and the airflow of the respiratory support device, while allowing the acoustic sensor to accurately detect the acoustic signal generated by the acoustic generator.

According to a first aspect, there is provided a respiratory support device for providing pressurized air to a subject. The respiratory support device comprises a conduit, an acoustic generator, and an acoustic sensor. The conduit is for conveying an airflow. The conduit has an opening. The acoustic generator is adapted to generate an acoustic signal in the conduit at an acoustic frequency. The acoustic sensor is arranged outside the conduit. The acoustic sensor is adapted to generate a sensor signal representative of the acoustic signal. The respiratory support device comprises a membrane arranged to cover the opening to separate the acoustic sensor and the airflow from each other. The membrane is adapted to resonate at the acoustic frequency.

The conduit is for conveying the airflow, and the acoustic sensor is arranged outside the conduit. Because the membrane is arranged to cover the opening in the conduit, the acoustic sensor is not exposed to the airflow, and any particles from the acoustic sensor are not able to enter the airflow in the conduit. The opening is arranged to allow the acoustic signal in the conduit to propagate via the opening to the acoustic sensor. Because the membrane is adapted to resonate at the acoustic frequency of the acoustic signal, the acoustic signal loses little or no energy when passing the membrane. As a result, the acoustic sensor receives sound that accurately represents the acoustic signal in the conduit. The acoustic sensor is able to generate the sensor signal that accurately represents the acoustic signal in the conduit. As a result, contamination between the acoustic sensor and the airflow is prevented, while the acoustic sensor is able to accurately detect the acoustic signal in the conduit generated by the acoustic generator.

For example, the respiratory support device is a PAP device or a ventilation device.

The conduit is formed, for example, at least partly by a hose or a channel in the respiratory support device. For example, one or more components inside the respiratory support device form at least part of the conduit. For example, the housing of the respiratory support device forms at least part of the conduit.

The acoustic generator is, for example, configured to sample or to receive at least part of the airflow. The acoustic generator is, for example, configured to generate the acoustic signal based on a characteristic of the airflow, such as pressure or flow speed. For example, the acoustic generator is adapted to generate the acoustic signal based on the airflow towards the subject. For example, the acoustic generator is adapted to generate the acoustic signal based on the airflow away from the subject.

The acoustic sensor is any sensor that is adapted to generate the sensor signal based on the acoustic signal. For example, the acoustic sensor is a microphone. For example, the acoustic sensor is a MEMS based microphone or an electret microphone.

The membrane is, for example, round shaped, or square shaped or rectangular shaped. The thickness of the membrane is substantially smaller than the length and/or width and/or diameter of the membrane to allow the membrane to resonate. For example, the thickness is in the range of 20-500 micrometer [$\mu$m], whereas the diameter or length or width are in the range of 1-5 mm. The membrane is airtight to prevent air from the conduit from reaching the acoustic sensor outside the conduit.

The membrane is adapted to resonate at the acoustic frequency. For example, the natural frequency of the membrane is in a range of +/-10% or +/- 20% of the acoustic frequency. For example, in case the acoustic frequency is 20 kHz, the natural frequency of the membrane is in the range of 16 kHz-24 kHz or in the range of 18 kHz- 22 kHz. For example, the amplitude of the vibration of the membrane is more than 10% or more than 20% higher at the acoustic frequency than at a frequency lower than the acoustic frequency.

In an embodiment, the acoustic generator is adapted to generate an inaudible acoustic signal. The acoustic frequency is in an inaudible acoustic frequency range.

According to this embodiment, a subject using the respiratory support device is not disturbed by the inaudible acoustic signal. This is especially important in case the respiratory support device is used by the subject during sleep. Despite being inaudible for the subject, the acoustic sensor is able to detect the inaudible acoustic signal and to generate the sensor signal.

In an embodiment, the inaudible acoustic frequency range is in the range of 16 kHz- 25 kHz.

According to this embodiment, the range of 16 kHz- 25 kHz creates acoustic signals that cannot be heard by most people. Various acoustic sensors are commercially available that are able to sense acoustic signals in this range. It is noted that some people, especially young people, may hear acoustic signals up to 20 kHz. However, most subjects that make use of a respiratory support device are middle aged people or elderly people, which cannot hear acoustic signals above 16 kHz. Further, also for people that are able to hear acoustic signals up to 20 kHz, the hearing threshold for sounds of 16 kHz and higher is substantially higher than the hearing threshold of sounds of less than 16 kHz. As a result, the acoustic signal is inaudible because the volume is lower than the hearing threshold at the range of 16 kHz- 25 kHz.

In an embodiment, the respiratory support device comprises an air chamber. The membrane is arranged to separate the air chamber and the airflow from each other. The acoustic sensor is arranged in the air chamber. The air chamber is dimensioned to cause air in the air chamber to act as a compliant spring. The membrane and the compliant spring form a dynamical system. The membrane is adapted to resonate at the acoustic frequency as part of the dynamical system.

According to this embodiment, an air chamber is used to form a compliant spring. The stiffness of the compliant spring and the stiffness of the membrane are part of the dynamical system that causes the membrane to resonate at the acoustic frequency. Without using the air chamber as the compliant spring, the membrane needs to have a very low mass and a very high stiffness to resonate at the acoustic frequency. By having the stiffness of the compliant spring and the stiffness of the membrane acting in parallel, the membrane may have a lower stiffness and/or a higher mass. This allows for more design freedom for the shape of the membrane, such as the thickness or length or width. This allows for more choice in materials because materials with a relatively low Youngs modulus and/or a high density may be selected. For example, the air chamber is substantially closed, except for a restricted opening to the environment. The restricted opening allows the pressure in the air chamber to change as a result of a change in the ambient pressure. Because of the restriction in the restricted opening, the change of pressure in the air chamber is at a low frequence, i.e., about 1 Hz or about 5 Hz. Because of this low frequence compared to the much higher acoustic frequency, the compliant spring in the air chamber is not affected by the restricted opening.

In an embodiment, the respiratory support comprises an air chamber. The membrane is arranged to separate the air chamber and the airflow from each other. The acoustic sensor is arranged in the air chamber. The acoustic sensor and the membrane are arranged at a distance from each other corresponding to half a wavelength of the acoustical frequency.

According to this embodiment, the distance between the acoustic sensor and the membrane corresponds to half the wavelength of the acoustical frequency. As a result, a vibration of the membrane at the acoustic frequency causes a standing wave of air in the air chamber. Because of the standing wave, a small vibration of the membrane causes a large pressure at the acoustic sensor. As a result, the acoustic sensor is able to accurately detect the acoustic signal.

In an embodiment, the distance is in a range of 6,88 - 10.75 mm.

According to this embodiment, half the wavelength of the acoustical frequency is in the range of 6,88 - 10.75 mm. The corresponding wavelengths are in the range of 13.76 - 21.50 mm. The speed of sound through air at room temperature is about 343 m/s. As a result, the distance relates to acoustic signals in the range of 16 kHz - 25 kHz.

In an embodiment, the membrane is pretensioned.

According to this embodiment, the membrane is pretensioned, i.e., tensile stress is applied to the membrane before the membrane is used to vibrate in response to the acoustic signal. Similarly to tuning a guitar, the natural frequency of the membrane is increased by applying the tensile stress. This allows for a high natural frequency of the membrane, without the need for a high stiffness of the membrane without pretension. For example, the membrane is pretensioned by mounting the membrane in a frame. The membrane is mounted to the frame while pretensioned. For example, the membrane is mounted in an adjustable frame. The adjustable frame has, for example, an adjustable length or an adjustable width. By increasing the length and/or the width of the adjustable frame while the membrane is mounted to the frame, the membrane is stretched and pretensioned. For example, the membrane is clamped or glued or soldered or welded to the frame, while the membrane is pretensioned.

In an embodiment, the respiratory support device comprises a control system. The membrane comprises an electroactive polymer and a control system. The electroactive polymer is adapted to deform in response to an electric field. The control system is configured to control a natural frequency of the membrane by controlling the electric field.

An electroactive polymer is a polymer that changes size or shape when stimulated by an electric field. By arranging the electroactive polymer on or in the membrane, the size and shape of the membrane is controlled by the control system. The control system is configured to control a voltage or an electric current provided to the electroactive polymer to change an electric field at the electroactive polymer. As a result, the size or shape of the membrane changes to a desired size or shape to obtain the desired natural frequency of the membrane. For example, the control system controls the electroactive polymer to stretch the membrane. As a result of stretching the membrane, the natural frequency of the membrane increases. For example, the control system controls the electroactive polymer to thicken the membrane. By thickening the membrane, the bending stiffness of the membrane increases. As a result of the increased bending stiffness, the natural frequency of the membrane increases.

For example, the control system is configured to control the electroactive polymer when the respiratory support device is not used by the subject, e.g., when the respiratory support device is not providing pressurized air. For example, the control system is configured to control the electroactive polymer when the respiratory support device is providing the pressurized air at a low pressure and does not control the electroactive polymer when the respiratory support device is providing the pressurized air at a low pressure. For example, the control system is configured to control the electroactive polymer when the respiratory support device is in use for a certain amount of time, such as more than half an hour or more than an hour.

In an embodiment, the membrane comprises at least a first material and a second material. The second material is a viscoelastic material having more hysteresis than the first material.

By using the viscoelastic material having the hysteresis, the membrane is provided with damping. Without any damping, the natural frequency membrane causes the membrane to resonate only in a small frequency band. By providing damping, the frequency band in which the membrane resonates is increased. This allows the membrane to resonate in response to a broader range of frequencies of the acoustic signal. For example, the acoustic generator may not generate the acoustic signal at exactly a desired acoustic frequency. This may be the result of manufacturing inaccuracies of the acoustic generator, or wear of the acoustic generator, or contamination of the acoustic generator. The membrane is still able to resonate because of the broader range of frequencies. For example, the respiratory support device has multiple acoustic generators. Each of the multiple acoustic generators generates an acoustic signal that has an acoustic frequency that is different from the others. For example, the difference between the acoustic signals is 50 Hz or 100 Hz or 200 Hz. Because of the broader range of frequencies, the membrane is able to resonate in response to any one of the acoustic signals. For example, the first material is steel or aluminum or titanium or platinum. For example, the second material is a plastic or a rubber or a silicone material or parafilm. For example, the second material is provided to the first material as a coating.

In an embodiment, the respiratory support device comprises a connector adapted to connect the membrane to the conduit. The membrane comprises at least the first material. The connector comprises at least a third material in contact with the membrane. The third material is a viscoelastic material having more hysteresis than the first material.

By using the viscoelastic material having the hysteresis, the membrane is provided with damping. Without any damping, the natural frequency membrane causes the membrane to resonate only in a small frequency band. By providing damping, the frequency band in which the membrane resonates is increased. This allows the membrane to resonate in response to a broader range of frequencies of the acoustic signal. For example, the acoustic generator may not generate the acoustic signal at exactly the desired acoustic frequency. This may be the result of manufacturing inaccuracies of the acoustic generator, or wear of the acoustic generator, or contamination of the acoustic generator. The membrane is still able to resonate because of the broader range of frequencies. For example, the respiratory support device has multiple acoustic generators. Each of the multiple acoustic generators generates an acoustic signal that has an acoustic frequency that is different from the others. For example, the difference between the acoustic signals is 50 Hz or 100 Hz or 200 Hz. Because of the broader range of frequencies, the membrane is able to resonate in response to any one of the acoustic signals. For example, the first material is steel or aluminum or titanium or platinum. For example, the third material is a plastic or a rubber or a silicone material or parafilm. For example, the connector is adapted to contact the membrane with the third material at an edge of the membrane. For example, the connector is adapted to contact the membrane with the third material at a plurality of locations at the edge of the membrane.

In an embodiment, the respiratory support device comprises a connector adapted to connect the membrane to the conduit. The connector comprises a compliant body adapted to couple the membrane and the conduit to each other. The membrane is adapted to resonate at the acoustic frequency in a rigid body resonance mode. In the rigid body resonance mode, a deformation of the membrane is smaller than a deformation of the compliant body.

According to this embodiment, the membrane resonates at the acoustic frequency by moving as a rigid body. The compliant body of the connector allows the membrane to move as a rigid body. During the rigid body resonance mode, the membrane may have some deformation, such as bending. However, this deformation is substantially less than the movement of the membrane allowed by the compliant body. For example, the deformation of the membrane is less than 70% or less than 50% or less than 30% or less than 10% than the movement of the membrane allowed by the compliant body.

In an embodiment, the respiratory support device comprises a patient interface adapted to provide the pressurized air to the subject. The acoustic generator is located adjacent to or in the patient interface.

According to this embodiment, the acoustic generator is located adjacent to or in the patient interface, which allows the acoustic generator to provide information about the patient interface. For example, the acoustic generator provides information about a status of the patient interface based on an airflow through the patient interface. For example, the acoustic generator provides information about a status of the subject based on an airflow through the patient interface, such as an airflow relating to inhalation by the subject or an airflow relating to exhalation by the subject.

In an embodiment, the acoustic generator comprises a whistle, a resonant pipe, a reed, a Helmholtz resonator, a further membrane, or a tensioned string. The acoustic generator is arranged at least partly inside the conduit to receive at least a portion of the airflow. The acoustic generator is adapted to generate the acoustic signal at the acoustic frequency dependent on a velocity of the airflow.

According to this embodiment, the acoustic generator is adapted to generate the acoustic signal in dependence of the velocity of the airflow through the conduit. This way, for example, the acoustic signal provides information about the breathing of the subject. This way, for example, the acoustic signal provides information about a leakage of air from the conduit. For example, an amplitude or a frequency of the acoustic signal depends on the velocity of the airflow. The frequency of the acoustic signal is within a range of frequencies at which the membrane is adapted to resonate.

In an embodiment, the acoustic generator comprises a windway and a blade formation. The windway has a windway inlet and a windway outlet. The windway inlet is adapted to receive a portion of the airflow. The blade formation is adjacent to the windway outlet and adapted to interact with the portion of airflow to produce a turbulent flow of air.

According to this embodiment, the windway is able to direct the portion of the airflow to the blade formation. The turbulent flow produced by the blade formation causes the acoustic signal. This way, the acoustic signal represents a property of the airflow.

According to a second aspect of the invention, there is provided a method for detecting an acoustic signal at an acoustic frequency in a conduit of a respiratory support device providing pressurized air to a subject. The method comprises conveying an airflow through the conduit having an opening. The method comprises generating the acoustic signal in the conduit at the acoustic frequency. The method comprises using a membrane to cover the opening to separate an acoustic sensor and the airflow from each other. The method comprises resonating the membrane at the acoustic frequency. The method comprises generating with the acoustic sensor a sensor signal representative of the acoustic signal.

BRIEF DESCRIPTION OF THE DRAWINGS

Exemplary embodiments will now be described, by way of example only, with reference to the following figures, in which:

FIG. 1 depicts a respiratory support device according to a first embodiment of the invention;
FIG. 2 depicts a detail of the first embodiment;
FIG. 3 depicts a second embodiment of the invention;
FIG. 4 depicts a dynamical system according to the second embodiment;
FIG. 5 depicts a third embodiment of the invention;
FIG. 6 depicts a frequency response function of the membrane;
FIG. 7 depicts a fourth embodiment of the invention;
FIG. 8 depicts a fifth embodiment of the invention;
FIGs. 9A and 9B depict an acoustic generator according to a sixth embodiment;
FIG. 10 depicts the acoustic generator according to a seventh embodiment;
FIG. 11 depicts the acoustic generator according to an eight embodiment;
FIG. 12 depicts a method according to a nineth embodiment.

DETAILED DESCRIPTION OF EMBODIMENTS

It should be understood that the detailed description and specific examples, while indicating exemplary embodiments of the respiratory support device, the acoustic generator, and the method, are intended for purposes of illustration only and are not intended to limit the scope of the invention. These and other features, aspects, and advantages of the respiratory support device, the acoustic generator, and the method of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

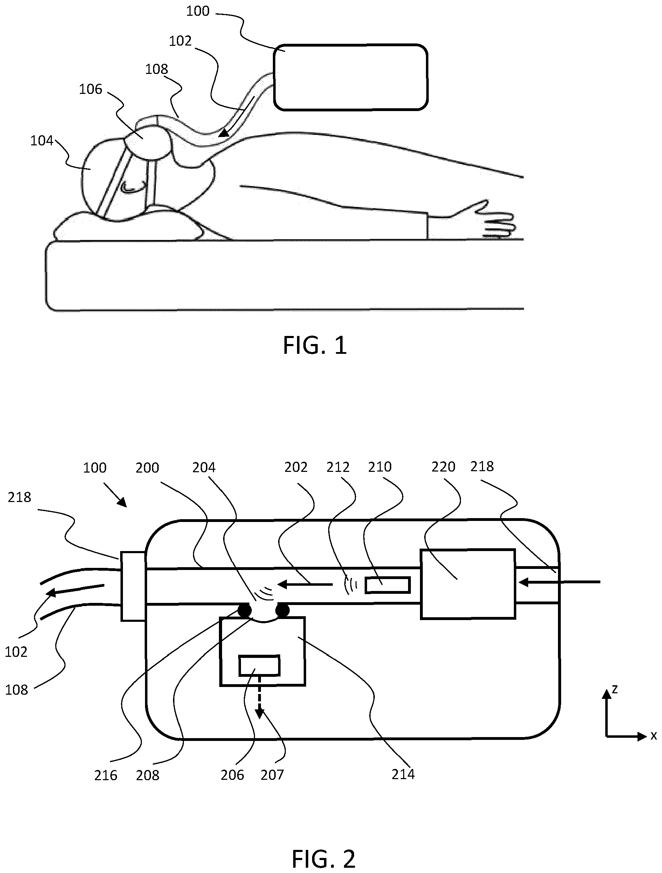

FIG. 1 depicts a respiratory support device 100 according to a first embodiment of the invention. The respiratory support device 100 is for providing pressurized air 102 to a subject 104. The respiratory support device 100 provides the pressurized air 102 via a delivery tube 108 to a patient interface 106. For example, the patient interface 106 is a nasal pillow, an oronasal mask or a full-face mask.

FIG. 2 depicts a detail of the first embodiment. The respiratory support device 100 comprises a conduit 200, an acoustic generator 210, an acoustic sensor 206, and a membrane 208. The conduit 200 is for conveying an airflow 202, such as the pressurized air 102. The conduit 200 has an opening 204. The acoustic generator 210 is adapted to generate an acoustic signal 212 in the conduit 200 at an acoustic frequency. The acoustic sensor 206 arranged outside the conduit 200. The acoustic sensor 206 is adapted to generate a sensor signal 207 representative of the acoustic signal 212. The membrane 208 is arranged to cover the opening 204 to separate the acoustic sensor 206 and the airflow 202 from each other. The membrane 208 is adapted to resonate at the acoustic frequency.

The respiratory support device 100 comprises an air chamber 214. The membrane 208 is arranged to separate the air chamber 214 and the airflow 202 from each other. The acoustic sensor 206 is arranged in the air chamber 214. A seal 216 is arranged between the conduit 200 and the air chamber 214 to prevent air from flowing out from between the conduit 200 and the air chamber 214.

The respiratory support device 100 comprises a pressure source 220 to generate the pressurized airflow 202 by pressurizing air received from an air inlet 216.

The acoustic generator 210 is adapted to generate an inaudible acoustic signal 212. The acoustic frequency is in an inaudible acoustic frequency range. For example, the inaudible acoustic frequency range is in the range of 16 kHz- 25 kHz.

For example, the membrane 208 is pretensioned.

FIG. 2 depicts the acoustic generator 210 arranged in the respiratory support device 100 between the inlet 218 and the delivery tube 108. In an embodiment, the patient interface 106 is adapted to provide the pressurized air 102 to the subject 104. The acoustic generator 210 is located adjacent to or in the patient interface 106.

Table 1 shows an example of the membrane 208 made from parafilm and an example of the membrane 208 made from aluminum. Parafilm is a blend of waxes and polyolefin. The examples are circular membranes. Based on the parameters, the natural frequency *f* is calculated by equation (1):

$$f = \frac{\alpha}{2\pi a^2} \sqrt{\frac{E h^2}{12(1-v^2)\rho}} \qquad (1)$$

The symbols of (1) are shown in table 1. The symbol $\alpha$ depends on the shape of the membrane and the size, and can be calculated according to formula's stated in Timoshenko, S., "Vibration problems in engineering", D. VAN NOSTRAND COMPANY, INC, New York, NY, USA, Second edition, 1937.

**Table 1: two examples of circular membranes**

| Symbol in (1) | Parameter | Parafilm | Aluminum |
|---|---|---|---|
| a | Radius [mm] | 2.5 | 2.5 |
| *h* | Thickness [μm] | 193 | 49.5 |
| *E* | Young's modulus [GPA] | 2 | 70 |
| *v* | Poisson's ratio [-] | 0.35 | 0.33 |
| *ρ* | Density [kg/m³] | 1200 | 2710 |
| *α* | Constant [-] | 10.21 | 10.21 |
| *f* | Natural frequency [kHz] | 20 | 20 |

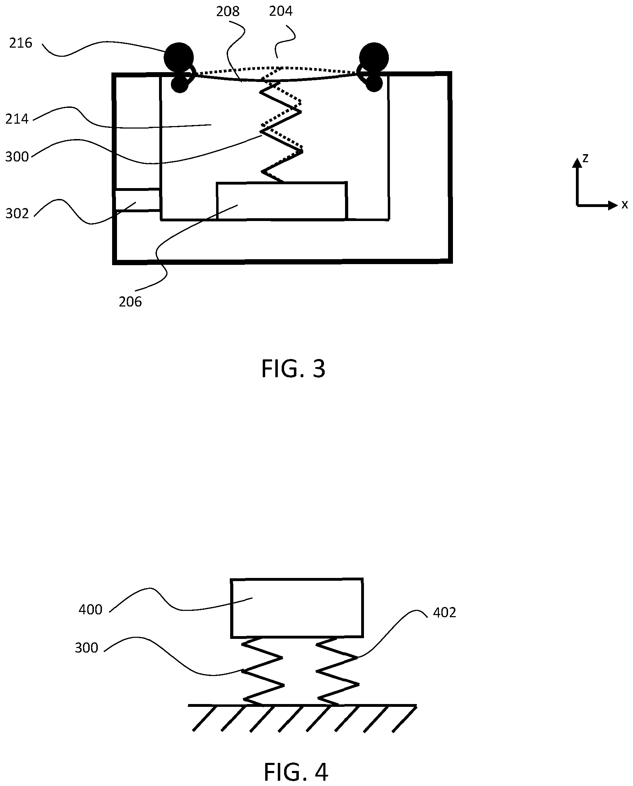

FIG. 3 depicts a second embodiment of the invention. The second embodiment has, for example, the same features as the first embodiment, except for the following.

The respiratory support device 100 comprises the air chamber 214. The membrane 208 is arranged to separate the air chamber 214 and the airflow 202 from each other. The acoustic sensor 206 is arranged in the air chamber 214. The air chamber 214 is dimensioned to cause air in the air chamber 214 to act as a compliant spring 300.

The membrane 208 and the compliant spring 300 form a dynamical system. The membrane 208 is adapted to resonate at the acoustic frequency as part of the dynamical system. The compliant spring 300 formed by the air chamber 214 is represented as a spring.

For example, the dimensions of the air chamber 214 are small compared in comparison to the wavelength of the acoustic frequency. A rule of thumb is that the dimensions of the air chamber 214 are smaller than 10% of the wavelength of the acoustic frequency. In case the acoustic frequency is in the range of 16 kHz- 25 kHz, the corresponding wavelengths are in the range of 13.76 - 21.50 mm. So the length of the air chamber 214 is in the range of 1-2 mm, as measured from the membrane 208 to the acoustic sensor 206.

Further, FIG. 3 depicts a restricted opening 302. The restricted opening 302 connects the air chamber 214 with the ambient environment. The restricted opening 302 allows the pressure in the air chamber 214 to change as a result of a change in the ambient pressure, such as caused by a change in weather. Because of the restriction in the restricted opening 302, the change of pressure in the air chamber 214 is at a low frequence, i.e., about 1 Hz or about 5 Hz. Because of this low frequence compared to the much higher acoustic frequency, the compliant spring 300 in the air chamber 214 is not affected.

FIG. 4 depicts the dynamical system according to the second embodiment. In the second embodiment, the membrane 208 and the compliant spring 300 form the dynamical system. The mass of the membrane 208 is represented by mass 400. The compliant spring 300 formed by the air chamber 214 is represented as spring. The bending stiffness of the membrane 208 is represented by the spring 402. The springs 300 and 402 act in parallel on the mass 400. As a result, the total stiffness with which mass 400 is connected to the fixed world is increased. With the increased total stiffness, the natural frequency of the membrane 208 is in the range of 16 kHz- 25 kHz.

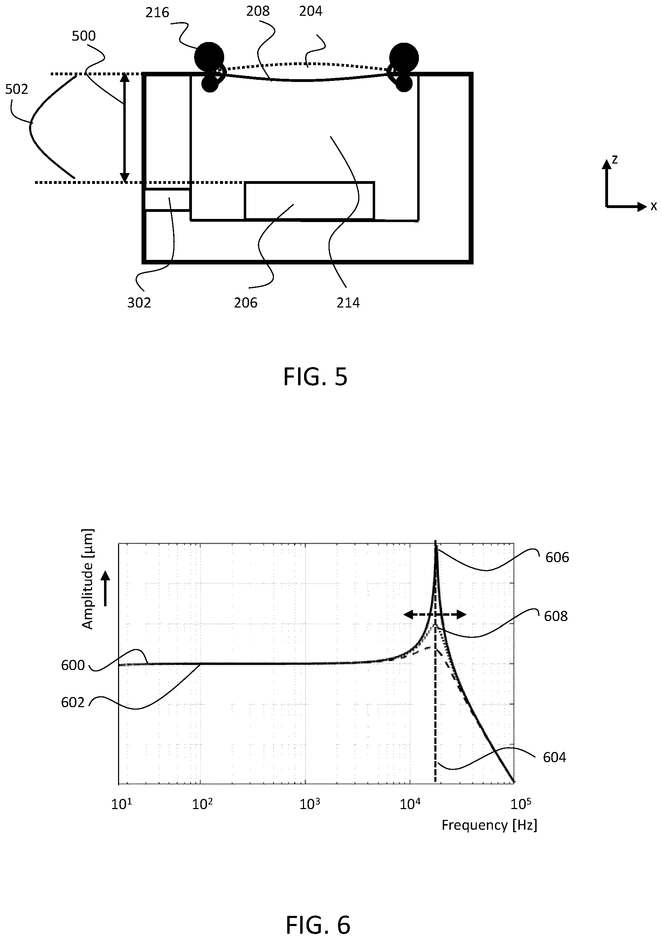

FIG. 5 depicts a third embodiment of the invention. The third embodiment has, for example, the same elements as the first embodiment or as the second embodiment, except for the following.

In the third embodiment, the respiratory support device 100 comprises an air chamber 214. The membrane 208 is arranged to separate the air chamber 214 and the airflow 202 from each other. The acoustic sensor 206 is arranged in the air chamber 214. The acoustic sensor 206 and the membrane 208 are arranged at a distance 500 from each other corresponding to half a wavelength 502 of the acoustical frequency. For example, the distance 500 is in a range of 6,88 - 10.75 mm.

Because the distance 500 corresponds to half the wavelength 502 of the acoustical frequency, a sound wave of the acoustic signal 212 has, for example, a node at the acoustic sensor 206 when the sound wave has a node at the membrane 208. For example, the sound wave has an anti-node at the acoustic sensor 206 when the sound wave has an anti-node at the membrane 208. The anti-node at the acoustic sensor 206 has a 180° phase difference with the anti-node at the membrane 208.

FIG. 6 depicts a frequency response function 600 of the membrane 208. The frequency response function 600 is similar for the first embodiment, for the second embodiment, and for the third embodiment. The frequency response function 600 depicts an amplitude of a vibration of the center of the membrane 208 along the z-axis as a function of the frequency exciting the membrane 208. The z-axis is perpendicular to the main plane of the membrane 208. The thickness of the membrane 208 is along the z-axis.

The frequency response function 600 shows that for frequencies up to about 4 kHz, the membrane 208 vibrates with a nominal amplitude 602. At frequencies near the natural frequency 604 of the membrane 208, the amplitude increases to a maximum 606 at the natural frequency 604.

The membrane 208 is adapted to resonate at the acoustic frequency. The membrane 208 is adapted to have the natural frequency 604 near the acoustic frequency. For example, the natural frequency 604 of the membrane 208 is in a range of +/-10% or +/- 20% of the acoustic frequency. For example, the membrane 208 resonates in response to the acoustic frequency in case the amplitude of the vibration of the membrane 208 is more than 10% or more than 20% higher at the acoustic frequency than the nominal amplitude 602.

In a fourth embodiment, damping is applied to the membrane 208. Damping causes the maximum amplitude 606 to reduce to a smaller maximum amplitude 608. Also, damping causes the resonance peak in the frequency response function 600 to broaden, as indicated with the double arrow. By broadening the resonance peak, the membrane 208 resonates for frequencies in a broader frequency range.

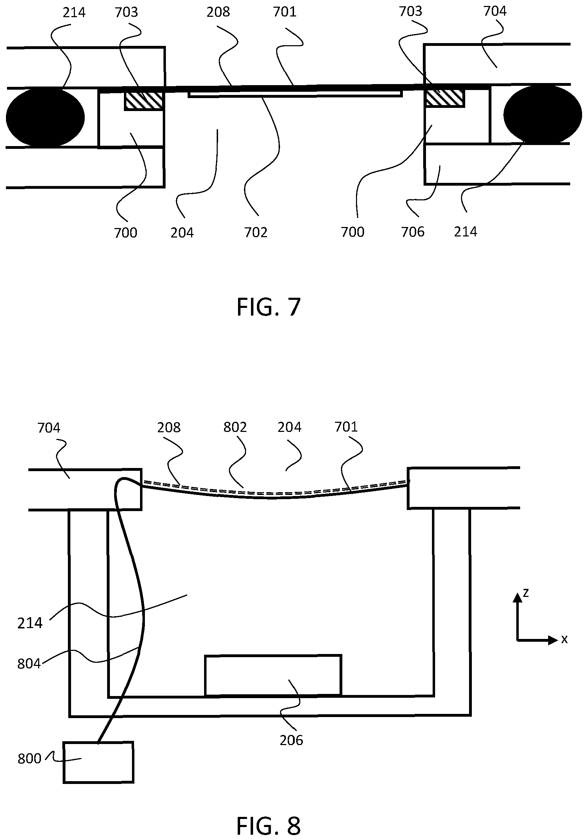

FIG. 7 depicts the fourth embodiment of the invention. The membrane 208 comprises at least a first material 701 and a second material 702. The second material 702 is a viscoelastic material having more hysteresis than the first material 701. For example, the first material 701 is steel or aluminum or titanium or platinum. For example, the second material 702 is a plastic or a rubber or a silicone material or parafilm. For example, the second material 702 is provided to the first material 701 as a coating. For example, the first material 701 is arranged between two layers of the second material 702. For example, the second material 702 covers an entire surface of the membrane 208 or only part of a surface of the membrane 208.

Further, FIG. 7 depicts the respiratory support comprising a connector 700. The connector 700 is adapted to connect the membrane 208 to the conduit 200. The membrane 208 comprises at least the first material 701. The connector 700 comprises at least a third material 703 in contact with the membrane 208. The third material 703 is a viscoelastic material having more hysteresis than the third material 703. For example, the third material 703 is a plastic or a rubber or a silicone material or parafilm. As shown in FIG. 7, the membrane 208 is coupled with the connector 700 to a wall 704 of the conduit 200. Another side of the connector 700 is coupled to a wall 706 of the air chamber 214. The third material 703 provides only part of the connection between the connector 700 and the wall 704 of the conduit 200 as is shown in FIG. 7. The other part of the connection between the connector 700 and the wall 704 of the conduit 200 is provided with another material than the third material 703. The desired amount of damping of the membrane 208 is determined by the amount or size or shape of the third material 703 in the connector 700. For example, the connector 700 comprises multiple bodies made from the third material 703, wherein each of the multiple bodies is in contact with the membrane 208.

In an embodiment, the membrane 208 comprises at least a first material 701 and a second material 702, whereas the connector 700 does not comprise the third material 703. In another embodiment, the connector 700 comprises the third material 703, whereas the membrane 208 does not comprise the first material 701 and the second material 702.

In an embodiment, the connector 700 is adapted to connect the membrane 208 to the conduit 200. The connector 700 comprises a compliant body adapted to couple the membrane 208 and the conduit 200 to each other. The membrane 208 is adapted to resonate at the acoustic frequency in a rigid body resonance mode. In the rigid body resonance mode, a deformation of the membrane 208 is smaller than a deformation of the compliant body.

FIG. 8 depicts a fifth embodiment of the invention. The fifth embodiment has, for example, the same elements as the first embodiment, or as the second embodiment, or as the third embodiment, or as the fourth embodiment, except for the following.

The respiratory support device 100 comprises a control system 800. The membrane 208 comprises an electroactive polymer 802 adapted to deform in response to an electric field. The control system 800 is configured to control a natural frequency of the membrane 208 by controlling the electric field.

A wire 804 connects the control system 800 with the electroactive polymer 802. The control system 800 is configured to provide an electric current or a voltage via the wire 804 to the electroactive polymer 802 to change the shape or the length or the width of the electroactive polymer 802 to change the natural frequency of the membrane 208.

For example, the membrane 208 comprises the first material 701. The electroactive polymer 802 is provided on or in the first material 701. For example, the electroactive polymer 802 is provided as a coating on the first material 701. For example, the electroactive polymer 802 covers an entire surface of the membrane 208. For example, the electroactive polymer 802 has the shape of a circle or a disk or a rectangle or a square. For example, the electroactive polymer 802 is provided as a pattern, such as a pattern of radially extending lines, or as a pattern of multiple concentric circles, or as a pattern of multiple squares, or as a pattern of multiple rectangles.

For example, the control system 800 is configured to receive the sensor signal 207. The control system 800 is, for example, configured to generate an output signal based on the sensor signal 207.

For example, the electroactive polymer 802 is a piezoelectric polymer. For example, the piezoelectric polymer is a polyvinylidene fluoride (PVDF), a poly(vinylidene fluoride-trifluoroethylene) (P(VDF-TrFE)), or a poly(vinylidene fluoride-trifluoroethylene-chlorofluoroethylene) (P(VDF-TrFE-CFE)). For example, the membrane 208 is provided with the electroactive polymer 802 on the surface of the membrane 208 facing the airflow 202, on the surface of the membrane 208 facing the acoustic sensor 206, or on both the surface of the membrane 208 facing the airflow 202 and the surface of the membrane 208 facing the acoustic sensor 206.

For example, the electroactive polymer 802 is an ionic polymer-metal composite (IPMC) or comprises a dielectric silicone.

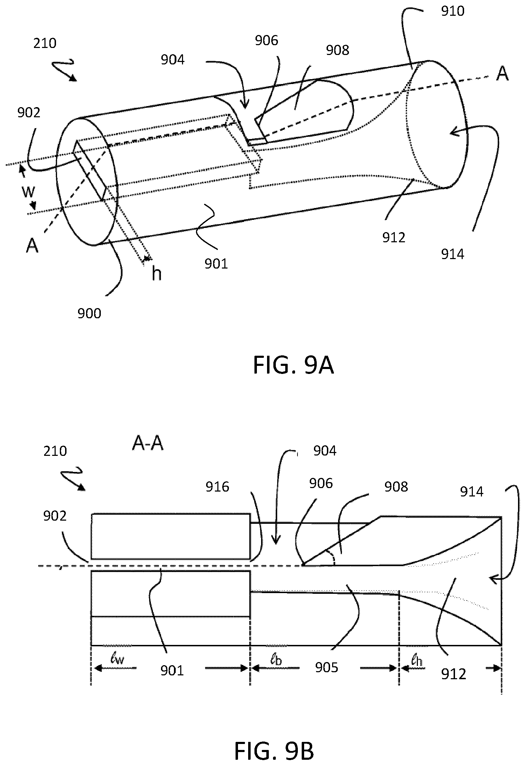

FIGs 9A and 9B depict the acoustic generator 210 according to a sixth embodiment. The acoustic generator 210 according to the sixth embodiment is, for example, used in the first embodiment, the second embodiment, the third embodiment, the fourth embodiment, or the fifth embodiment. The acoustic generator 210 is configured as a transducer for generating the acoustic signal 212. FIG. 9B depicts a cross sectional view of the acoustic generator 210 taken alone dotted line A-A of FIG. 9A. The acoustic generator 210 has an outer surface 900. A portion of the airflow 202 passes the acoustic generator 210 along the outer surface 900. The acoustic generator 210 has a windway 901. The windway 901 has a windway inlet 902 and a windway outlet 916. Another portion of the airflow 202 enters the windway 901 via the windway inlet 902 and travels towards the windway outlet 916. The windway outlet 916 is in fluid communication with a vortex outlet 904 and a resonance chamber 905. A blade formation 908 having a blade leading edge 906 is located in the vortex outlet 904. The portion of the airflow 202 exiting via the windway outlet 916 moves above and below the blade formation 908, which causes the blade leading edge 906 to oscillate. The oscillation generates the acoustic signal 212 by the generation of vortices within the vortex outlet 904. The portion of the airflow 202 that moves below the blade formation 908 enters the resonance chamber 905. The portion of the airflow 202 exists the acoustic generator 210 via an outlet 914.

The acoustic generator 210 comprises the windway 901 having the windway inlet 902 and the windway outlet 916. The windway inlet 902 is adapted to receive a portion of the airflow 202. The blade formation 908 is adjacent to the windway outlet 916. The blade formation 908 is adapted to interact with the portion of airflow 202 to produce a turbulent flow of air.

In an embodiment, the acoustic generator 210 comprises a whistle, a resonant pipe, a reed, a Helmholtz resonator, a further membrane, or a tensioned string. The acoustic generator 210 is arranged at least partly inside the conduit 200 to receive at least a portion of the airflow 202. The acoustic generator 210 is adapted to generate the acoustic signal 212 at the acoustic frequency dependent on a velocity of the airflow 202.

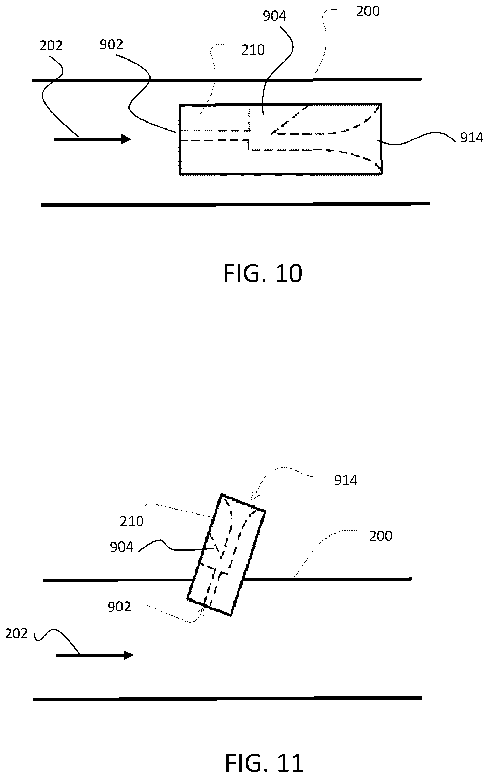

FIG. 10 depicts the acoustic generator 210 in a seventh embodiment. The acoustic generator 210 according to the seventh embodiment is, for example, the same as the acoustic generator 210 according to the sixth embodiment, except for the following. The acoustic generator 210 according to the seventh embodiment is, for example, used in the first embodiment, the second embodiment, the third embodiment, the fourth embodiment, or the fifth embodiment.

In the seventh embodiment, the acoustic generator 210 is arranged in the conduit 200. The windway inlet 902, the vortex outlet 904, and the outlet 914 are arranged inside the conduit 200. As a result, no portion of the airflow 202 leaves the conduit 200 via the acoustic generator 210.

FIG. 11 depicts the acoustic generator 210 in an eighth embodiment. The acoustic generator 210 according to the eighth embodiment is, for example, the same as the acoustic generator 210 according to the sixth embodiment, except for the following. The acoustic generator 210 according to the eighth embodiment is, for example, used in the first embodiment, the second embodiment, the third embodiment, the fourth embodiment, or the fifth embodiment.

In the eighth embodiment, the acoustic generator 210 is arranged partly inside the conduit 200 and partly outside the conduit 200. The acoustic generator 210 is arranged to have the windway inlet 902 inside the conduit 200. This way, the windway inlet 902 is in fluid communication with the airflow 202 in the conduit 200. The vortex outlet 904 and the outlet 914 are arranged outside the conduit 200. Preferably, only a small portion of the airflow 202 flows via the acoustic generator 210 out of the conduit 200. This way, a large portion of the airflow 202 remains in the conduit 200. For example, in case the airflow 202 is exhaled by the subject 104, a substantial portion of the airflow 202 may flow via the acoustic generator 210 out of the conduit 200.

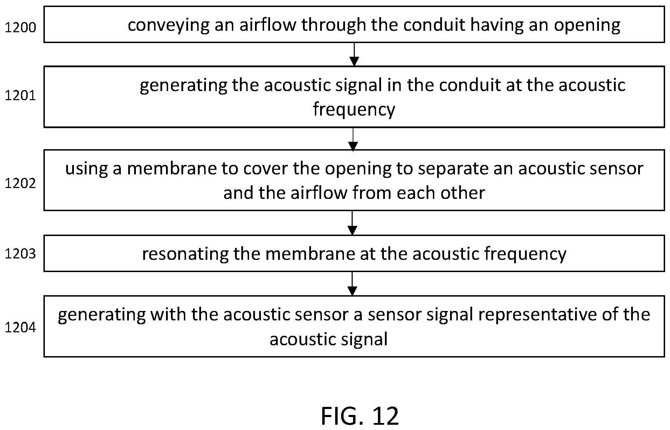

FIG. 12 depicts a method according to a nineth embodiment. The method according to the nineth embodiment is, for example, performed by the respiratory support device 100 according to the first embodiment, the second embodiment, the third embodiment, the fourth embodiment, or the fifth embodiment. The method according to the nineth embodiment makes use of, for example, the acoustic generator 210 according to the sixth embodiment or the seventh embodiment or the eighth embodiment.

The method according to the nineth embodiment is a method for detecting the acoustic signal 212 at the acoustic frequency in the conduit 200 of the respiratory support device 100. The respiratory support device 100 provides pressurized air 102 to the subject 104. The method comprises, in 1200, conveying the airflow 202 through the conduit 200 having the opening 204. The method comprises, in 1201, generating the acoustic signal 212 in the conduit 200 at the acoustic frequency. The method comprises, in 1202, using the membrane 208 to cover the opening 204 to separate the acoustic sensor 206 and the airflow 202 from each other. The method comprises, in 1203, resonating the membrane 208 at the acoustic frequency. The method comprises, in 1204, generating with the acoustic sensor 206 the sensor signal 207 representative of the acoustic signal 212.

The control system 800, as discussed above, may make use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Functions implemented by a processor may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A respiratory support device (100) for providing pressurized air (102) to a subject (104), the respiratory support device (100) comprising:

   a conduit (200) for conveying an airflow (202),
   wherein the conduit (200) has an opening (204);
   an acoustic generator (210) adapted to generate an acoustic signal (212) in the conduit (200) at an acoustic frequency;
   an acoustic sensor (206) arranged outside the conduit (200),
   wherein the acoustic sensor (206) is adapted to generate a sensor signal (207) representative of the acoustic signal (212);
   a membrane (208) arranged to cover the opening (204) to separate the acoustic sensor (206) and the airflow (202) from each other;
   wherein the membrane (208) is adapted to resonate at the acoustic frequency.

2. The respiratory support device (100) according to claim 1, wherein the acoustic generator (210) is adapted to generate an inaudible acoustic signal (212), wherein the acoustic frequency is in an inaudible acoustic frequency range.

3. The respiratory support device (100) according to claim 2, wherein the inaudible acoustic frequency range is in the range of 16 kHz- 25 kHz.

4. The respiratory support device (100) according to any one of the preceding claims, comprising an air chamber (214),

   wherein the membrane (208) is arranged to separate the air chamber (214) and the airflow (202) from each other;
   wherein the acoustic sensor (206) is arranged in the air chamber (214);
   wherein the air chamber (214) is dimensioned to cause air in the air chamber (214) to act as a compliant spring (300),
   wherein the membrane (208) and the compliant spring (300) form a dynamical system,
   wherein the membrane (208) is adapted to resonate at the acoustic frequency as part of the dynamical system.

5. The respiratory support device (100) according to any one of claims 1-3, comprising an air chamber (214),

   wherein the membrane (208) is arranged to separate the air chamber (214) and the airflow (202) from each other;
   wherein the acoustic sensor (206) is arranged in the air chamber (214);
   wherein the acoustic sensor (206) and the membrane (208) are arranged at a distance (500) from each other corresponding to half a wavelength of the acoustical frequency.

6. The respiratory support device (100) according to claim 5, wherein the distance (500) is in a range of 6,88 - 10.75 mm.

7. The respiratory support device (100) according to any one of the preceding claims, wherein the membrane (208) is pretensioned.

8. The respiratory support device (100) according to any one of the preceding claims, comprising a control system (800),

   wherein the membrane (208) comprises an electroactive polymer (802) adapted to deform in response to an electric field,
   wherein the control system (800) is configured to control a natural frequency of the membrane (208) by controlling the electric field.

9. The respiratory support device (100) according to any one of the preceding claims, wherein the membrane (208) comprises at least a first material (701) and a second material (702),
wherein the second material (702) is a viscoelastic material having more hysteresis than the first material (701).

10. The respiratory support device (100) according to any one of the preceding claims, comprising a connector (700) adapted to connect the membrane (208) to the conduit (200),

wherein the membrane (208) comprises at least a first material (701),
wherein the connector (700) comprises at least a third material (703) in contact with the membrane (208),
wherein the third material (703) is a viscoelastic material having more hysteresis than the third material (703).

11. The respiratory support device (100) according to any one of the preceding claims, comprising a connector (700) adapted to connect the membrane (208) to the conduit (200),

wherein the connector (700) comprises a compliant body adapted to couple the membrane (208) and the conduit (200) to each other,
wherein the membrane (208) is adapted to resonate at the acoustic frequency in a rigid body resonance mode,
wherein, in the rigid body resonance mode, a deformation of the membrane (208) is smaller than a deformation of the compliant body.

12. The respiratory support device (100) according to any one of the preceding claims, comprising a patient interface (106) adapted to provide the pressurized air (102) to the subject (104),
wherein the acoustic generator (210) is located adjacent to or in the patient interface (106).

13. The respiratory support device (100) according to any one of the preceding claims, wherein the acoustic generator (210) comprises a whistle, a resonant pipe, a reed, a Helmholtz resonator, a further membrane, or a tensioned string,

wherein the acoustic generator (210) is arranged at least partly inside the conduit (200) to receive at least a portion of the airflow (202),
wherein the acoustic generator (210) is adapted to generate the acoustic signal (212) at the acoustic frequency dependent on a velocity of the airflow (202).

14. The respiratory support device (100) according to any one of the preceding claims, wherein the acoustic generator (210) comprises:

a windway (901) having a windway inlet (902) and a windway outlet (916),
wherein the windway inlet (902) is adapted to receive a portion of the airflow (202);
a blade formation (908) adjacent to the windway outlet (916) and adapted to interact with the portion of airflow (202) to produce a turbulent flow of air.

15. A method for detecting an acoustic signal (212) at an acoustic frequency in a conduit (200) of a respiratory support device (100) providing pressurized air (102) to a subject (104), the method comprising:

conveying (1200) an airflow (202) through the conduit (200) having an opening (204);
generating (1201) the acoustic signal (212) in the conduit (200) at the acoustic frequency;
using (1202) a membrane (208) to cover the opening (204) to separate an acoustic sensor (206) and the airflow (202) from each other;
resonating (1203) the membrane (208) at the acoustic frequency;
generating (1204) with the acoustic sensor (206) a sensor signal (207) representative of the acoustic signal (212).

FIG. 1

FIG. 2

FIG. 3

FIG. 4

FIG. 5

FIG. 6

FIG. 7

FIG. 8

210

902

904    906    908    910

w

h

A

A

901

900

**FIG. 9A**

212

914

A-A

210

916    904

902    906    908    914

$\ell_w$    901    $\ell_b$    905    $\ell_h$    912

**FIG. 9B**

FIG. 10

FIG. 11

| 1200 | conveying an airflow through the conduit having an opening |
|---|---|
| 1201 | generating the acoustic signal in the conduit at the acoustic frequency |
| 1202 | using a membrane to cover the opening to separate an acoustic sensor and the airflow from each other |
| 1203 | resonating the membrane at the acoustic frequency |
| 1204 | generating with the acoustic sensor a sensor signal representative of the acoustic signal |

FIG. 12

Europäisches
Patentamt

European
Patent Office

Office européen
des brevets

# EUROPEAN SEARCH REPORT

**Application Number**

EP 24 18 5953

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/207796 A1 (RESMED PTY LTD [AU]) 21 October 2021 (2021-10-21) | 1,7,11, 12,15 | INV. G01F1/66 |
| Y | * paragraph [0405] * | 2,3,13, | G01F15/16 |
|  | * paragraph [0449] * | 14 | A61M16/00 |
| A | * paragraph [0511] * | 4-6,8-10 |  |
|  | * paragraph [0522] * |  | ADD. |
|  | * figures 1a,1b,1c,38-44 * |  | A61M16/08 |
|  | * claim 1 * |  |  |
|  | ----- |  |  |
| Y,D | US 2020/282161 A1 (CHAMTIE HAYAT [AU] ET AL) 10 September 2020 (2020-09-10) | 2,3,13, 14 |  |
|  | * paragraph [0246] - paragraph [0251] * |  |  |
|  | * claims 23-29 * |  |  |
|  | * figures 7a-7h * |  |  |
|  | ----- |  |  |
| A | AU 2013 201 312 A1 (RESMED LTD) 21 March 2013 (2013-03-21) | 1,4,15 |  |
|  | * paragraph [0090] * |  |  |
|  | * paragraph [0130] * |  |  |
|  | * figure 1 * |  |  |
|  | ----- |  | TECHNICAL FIELDS SEARCHED (IPC) |
| A | EP 3 632 491 A1 (RESMED PTY LTD [AU]) 8 April 2020 (2020-04-08) | 1,15 | G01F A61M |
|  | * paragraph [0065] * |  |  |
|  | * paragraph [0087] * |  |  |
|  | * figures 7,13 * |  |  |
|  | ----- |  |  |
| A | US 2016/199607 A1 (KENYON BARTON JOHN [AU] ET AL) 14 July 2016 (2016-07-14) | 8-10 |  |
|  | * paragraph [0143] - paragraph [0151] * |  |  |
|  | * figures 6a-6n * |  |  |
|  | ----- |  |  |

The present search report has been drawn up for all claims

| Place of search | Date of completion of the search | Examiner |
|---|---|---|
| The Hague | 29 November 2024 | Verdoodt, Erik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 18 5953

This annex lists the patent family members relating to the patent documents cited in  the above-mentioned European search report.
The members are as contained in the European Patent Office EDP file on
The European Patent Office is in no way liable for these particulars which are merely given for the purpose of information.

29-11-2024

| Patent document cited in search report | Publication date | Patent family member(s) | | Publication date |
|---|---|---|---|---|
| WO 2021207796 A1 | 21-10-2021 | AU | 2021255142 A1 | 10-11-2022 |
| | | CN | 115916308 A | 04-04-2023 |
| | | CN | 216148035 U | 01-04-2022 |
| | | EP | 4135806 A1 | 22-02-2023 |
| | | JP | 7571152 B2 | 22-10-2024 |
| | | JP | 2023522204 A | 29-05-2023 |
| | | US | 2023158266 A1 | 25-05-2023 |
| | | WO | 2021207796 A1 | 21-10-2021 |
| US 2020282161 A1 | 10-09-2020 | EP | 3648823 A1 | 13-05-2020 |
| | | EP | 4209240 A1 | 12-07-2023 |
| | | US | 2020282161 A1 | 10-09-2020 |
| | | US | 2023381435 A1 | 30-11-2023 |
| | | WO | 2019006496 A1 | 10-01-2019 |
| AU 2013201312 A1 | 21-03-2013 | NONE | | |
| EP 3632491 A1 | 08-04-2020 | AU | 2010213352 A1 | 11-08-2011 |
| | | CN | 102316919 A | 11-01-2012 |
| | | CN | 106214176 A | 14-12-2016 |
| | | CN | 110975088 A | 10-04-2020 |
| | | EP | 2396062 A1 | 21-12-2011 |
| | | EP | 3632491 A1 | 08-04-2020 |
| | | EP | 4241673 A2 | 13-09-2023 |
| | | JP | 5738201 B2 | 17-06-2015 |
| | | JP | 6055507 B2 | 27-12-2016 |
| | | JP | 2012517303 A | 02-08-2012 |
| | | JP | 2015180255 A | 15-10-2015 |
| | | NZ | 594185 A | 20-12-2013 |
| | | NZ | 612275 A | 28-03-2014 |
| | | NZ | 617502 A | 29-05-2015 |
| | | NZ | 707067 A | 25-11-2016 |
| | | US | 2011313689 A1 | 22-12-2011 |
| | | US | 2020114100 A1 | 16-04-2020 |
| | | US | 2020368469 A1 | 26-11-2020 |
| | | WO | 2010091462 A1 | 19-08-2010 |
| US 2016199607 A1 | 14-07-2016 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200282161 A1